# EUROPEAN PATENT SPECIFICATION

(11) **EP 3 130 649 B1**
(45) Date of publication and mention of the grant of the patent: **27.11.2024**
(21) Application number: 15190792.0
(22) Date of filing: 10.08.2015
(51) Int. Cl.: C09K 5/04, C07C 21/18, C10M 171/00

(54) **HALOOLEFIN-BASED COMPOSITION**
HALOOLEFINBASIERTE ZUSAMMENSETZUNG
COMPOSITION À BASE D'HALOGÉNOOLÉFINE

(30) Priority: 26.09.2014 JP 2014196449
(43) Date of publication of application: 15.02.2017
(62) Divisional of application: 15760361.4
(73) Proprietor: DAIKIN INDUSTRIES, LTD., Osaka-Shi, Osaka 530-0001 (JP)
(72) Inventor: Takahashi, Kazuhiro, Osaka, 5300001 (JP); Tsuchiya, Tatsumi, Osaka, 5300001 (JP); Yamada, Yasufu, Osaka, 5300001 (JP)
(74) Representative: Hoffmann Eitle

(56) References cited:
- US-A1- 2011 144 216
- US-A1- 2012 068 105
- US-A1- 2012 255 316
- US-A1- 2013 099 154
- US-A1- 2014 070 129
- US-A1- 2014 305 161
- "excerpt from the Römpp Chemistry Encyclopedia, 10th Edition (1996) for the keyword "polyalkylene glycols", 1 January 1996
- "Datasheet for the Polyol Ester Ze-GLES RB", 1 January 2018
- NGOC DUNG (ROSIE) ROHATGI ET AL: "Material compatibility & lubricants research for low gwp refrigerants - phase I: Thermal and chemical stability of low gwp refrigerants with lubricants", AHRTI FINAL REPORT 09004-01, 1 March 2012 (2012-03-01), pages 1 - 62, XP055460719
- ANONYMOUS: "Specifications for Fluorocarbon Refrigerants", AHRI STANDARD 700, 1 January 2012 (2012-01-01), pages 1 - 17, XP055460691
- FLEEMAN TAMMYE L: "AN INHALATION PRENATAL DEVELOPMENTAL TOXICITY STUDY OF HFO-1234yf (2,3,3,3-TETRAFLUOROPROPENE) IN RABBITS", 16 November 2009 (2009-11-16), pages 1 - 480, XP055979282, Retrieved from the Internet <URL:https://downloads.regulations.gov/EPA-HQ-OAR-2008-0664-0041/content.pdf> [retrieved on 20221108]

## Description

### Technical Field

The present invention relates to a haloolefin-based composition.

### Background Art

Hydrofluorocarbons (HFCs), such as HFC-125 and HFC-32, have been widely used as important substitutes for e.g. CFCs and HCFCs, which are known as substances that deplete the ozone layer. Known examples of such substitutes include "HFC-410A," which is a mixture of HFC-32 and HFC-125, and "HFC-404A," which is a mixture of HFC-125, HFC-134a and HFC-143a.

Such substitutes have various applications, such as heat transfer media, refrigerants, foaming agents, solvents, cleaning agents, propellants, and fire extinguishers, and are consumed in large amounts. However, since these substances have a global warning potential (GWP) several thousand times higher than that of CO₂, many people are concerned that their diffusion may affect global warming. As a global warming countermeasure, the substances are collected after being used; however, not all of them can be collected, and their diffusion due to e.g. leakage, cannot be disregarded. For use in e.g. refrigerants and heat transfer media, although substitution with CO₂ or hydrocarbon-based substances has been studied, CO₂ refrigerants have many difficulties in reducing comprehensive greenhouse gas emissions, including energy consumption, because of the requirement of large equipment due to the low efficiency of the CO₂ refrigerants. Hydrocarbon-based substances also pose safety problems due to their high flammability.

Hydrofluoroolefins with a low warming potential are recently attracting attention as substances that can solve these problems. Hydrofluoroolefin is a generic name for unsaturated hydrocarbons containing hydrogen, fluorine, and chlorine, and includes substances represented by the following chemical formulae. The description in parentheses following each chemical formula indicates the refrigerant number typically used for refrigerant purposes.
CF₃CF=CF₂ (FC-1216 or hexafluoropropene),
CF₃CF=CHF (HFO-1225ye),
CF₃CF=CH₂ (HFO-1234yf),
CF₃CH=CHF (HFO-1234ze),
CF₃CH=CH₂ (HFO-1243zf)
CF₃CCl=CH₂ (HCFO-1233xf),
CF₂ClCCl=CH₂ (HCFO-1232xf),
CF₃CH=CHCl (HCFO-1233zd),
CF₃CCl=CHCl (HCFO-1223xd),
CClF₂CCl=CHCl (HCFO-1222xd),
CFCl₂CCl=CH₂ (HCFO-1231xf), and
CH₂ClCCl=CCl₂ (HCO-1230xa).

Of these, fluoropropenes are particularly promising substances as candidates for refrigerants or heat transfer media with a low GWP; however, they may, for example, sometimes gradually decompose over time, and thus are not highly stable. Accordingly, these substances have a problem of gradually reducing performance depending on the situation or environment when used in various applications.

To enhance the stability of fluoropropenes, a method for adding a phenol compound to a composition containing HFO-1234yf and CF₃I is known (see, for example, WO2005/103187).

EP-A-2 432 441 discloses a composition comprising at least one lubricant made of polyol esters (POE) or polyvinyl ether (PVE) and a refrigerant F comprising 1-99 wt.% of 2,3,3,3-tetrafluoropropene (HFO-1234yf) and 1-99 wt.% of trans-1,3,3,3-tetrafluoropropene (trans-HFO-1234ze).

WO 2013/099856 (corresponding to EP-A-2 799 415) describes a method for continuously purifying crude HFO-1234yf containing water and one or more organic impurities, which method includes using an apparatus having a distillation column with X stages (3 ≤ X, the stage closest to a column top is the first stage) and a unit for cooling and condensing a distillate; supplying crude HFO-1234yf to an m-th stage ((n+1) ≤ *m* ≤ X, 2 ≤ *n* ≤ (X-1)) of the distillation column; recirculating at least part of the distillate cooled and condensed in the unit for cooling and condensing to an h-th stage ((1 ≤ *h* ≤ (n-1)) of the distillation column; and taking out a liquid phase part of an n-th stage of the distillation column to obtain a purified product of HFO-1234yf having low contents of organic impurities and water.

EP-A-2 591 061 relates to heat transfer fluid combinations of 1,3,3,3-tetrafluoropropene (HFO-1234ze) and polyol ester (POE), which may contain a variety of other refrigerant components and preferably has a low water content.

EP-A-2 513 244 discloses heat exchange method performed in a steam-compression circuit containing a heat-transfer fluid (such as combinations of HFO-1234yf or HFO-1234ze with any of HFC-32, HFC-125 and HFC-134a) and being at least partially contained in an enclosure, wherein the relative humidity of the air is reduced to lower the flammability of the heat-transfer fluid.

EP-A-2 709 972 describes a composition which is useful as e.g. refrigerant or heat transfer fluid, and which comprises (i) l-chloro-3,3,3-trifluoropropene and (ii) 0.001-5 wt.-% in total of HFO-1234ze and/or 3,3,3-trifluoropropene, where the vapor of the composition is non-flammable at at least one temperature of 20-100°C when mixed with at least one concentration in the range of 3-22 vol.-% of air having a relative humidity of 50%.

EP-A_-2 513 023 discloses compositions comprising cis-1,1,1,4,4,4-hexafluoro-2-butene and at least one additional compound selected from a broad variety of alternatives including HFOs (such as HFO-1234ze) and HFCs.

### Summary of Invention

### Technical Problem

The above method of WO 2005/103187 can improve the stability of HFO-1234yf by the effect of the phenol compound; however, it still has a problem of handling difficulty during mixing. The method for improving stability by adding a phenol compound as described above may also reduce the performance of fluoropropenes by the effect of the phenol compound, and has a problem in improving stability while maintaining performance.

The present invention was accomplished based on the above, and an object of the present invention is to provide a highly stable haloolefin-based composition that inhibits decomposition or oxidization.

### Solution to Problem

As a result of extensive research to achieve the above object, the present inventors found that the above object can be attained by containing a very small amount of water in a mixture containing a haloolefin and other specific substances, and accomplished the invention. Specifically, the present invention relates to a haloolefin-based composition comprising
(a) a haloolefin selected from tri-, tetra- and pentafluoropropene;
(b) at least one compound selected from HFO-1234zeHFC-254eb, HFC-245eb, HFC-245fa, HFC-245cb, HFC-236ea, HFC-236fa, HFC-23, HFC-32, HFC-125, HFC-143a, HFC-134a, FC-1216, HCFO-1233xf, HCFO-1233zd, HCFO-1232xf, HCFO-1223xd, chloromethane and, if the haloolefin (a) is HFO-1234ze, HFO-1234yf;
(c) water; and
(d) oxygen,

wherein the amount of HFO-1243zf, HFO-1225ye, 3,3,3-trifluoropropine and the at least one compound (b) is 0.1 to < 10,000 ppm by weight based on the total amount of the haloolefin (a),
the amount of water (c)being 3-200 ppm by weight based on the total amount of the haloolefin (a),
the amount of oxygen (d) being ≤ 0.35 mol% based on the total amount of the haloolefin (a), and
the component (b) being different from the haloolefin (a), and wherein the composition does not comprise polyalkyleneglycol (PAG) or polyolester (POE).

Preferred embodiments of the composition of the invention are as defined in the appended dependent claims and/or the following detailed description.

### Advantageous Effects of Invention

According to the haloolefin-based composition of the present invention, the stability of haloolefin in the composition is improved because the composition contains water. Specifically, since the double bond in the molecule of the haloolefin can be stably present, and the haloolefin does not easily cause oxidization, the performance of the haloolefin is not likely to be lost for a long period of time.

### Description of Embodiments

Hereinafter, the present invention is explained in detail.

The haloolefin-based composition comprises
(a) a haloolefin selected from tri-, tetra- and pentafluoropropene;
(b) at least one compound selected from HFO-1234ze, HFC-254eb, HFC-245eb, HFC-245fa, HFC-245cb, HFC-236ea, HFC-236fa, HFC-23, HFC-32, HFC-125, HFC-143a, HFC-134a, FC-1216, HCFO-1233xf, HCFO-1233zd, HCFO-1232xf, HCFO-1223xd, chloromethane and, if the haloolefin (a) is HFO-1234ze, HFO-1234yf;
(c) water; and
(d) oxygen,

wherein the amount of HFO-1243zf, HFO-1225ye, 3,3,3-trifluoropropine and the at least one compound (b) is 0.1 to < 10,000 ppm by weight based on the total amount of the haloolefin (a),
the amount of water (c)being 3-200 ppm by weight based on the total amount of the haloolefin (a),
the amount of oxygen (d) being ≤ 0.35 mol% based on the total amount of the haloolefin (a), and
   the component (b) being different from the haloolefin (a), and wherein the composition does not comprise polyalkyleneglycol (PAG) or polyolester (POE).

HFO-1234ze is 1,3,3,3-tetrafluoropropene, HFC-254eb is 1,1,1,2-tetrafluoropropane, HFO-1243zf is 3,3,3-trifluoropropene, HFC-245eb is 1,1,1,2,3-pentafluoropropane, HFC-245fa is 1,1,1,3,3-pentafluoropropane, HFC-245cb is 1,1,1,2,2-pentafluoropropane, HFC-236ea is 1,1,1,2,3,3-hexafluoropropane, HFC-236fa is 1,1,1,3,3,3-hexafluoropropane, HFO-1225ye is 1,2,3,3,3-pentafluoropropene, HFC-23 is trifluoromethane, HFC-32 is methylene difluoride, HFC-125 is 1,1,1,2,2-pentafluoroethane, HFC-143a is 1,1,1-trifluoroethane, HFC-134a is 1,1,1,2-tetrafluoroethane, FC-1216 is hexafluoropropene, HCFO-1233xf is 2-chloro-3,3,3-trifluoropropene, HCFO-1233zd is 1-chloro-3,3,3-trifluoropropene, HCFO-1232xf is 3,3-difluoropropene, and HCFO-1223xd is 1,2-dichloro-3,3,3-trifluoropropene.

The component (b) is a byproduct obtained in the production of the (haloolefin a). Accordingly, the component (b) is different from the haloolefin (a).

Because the haloolefin-based composition (hereinbelow, sometimes referred to as a "composition") contains water, the double bond in the molecule of the haloolefin can be stably present, and oxidization of the haloolefin does not easily occur. As a result, the stability of the haloolefin is improved.

The haloolefin is an unsaturated hydrocarbon selected from tetrafluoropropene, pentafluoropropene, and trifluoropropene. The isomer types of these compounds are not particularly limited. Particularly preferable examples include 2,3,3,3-tetrafluoropropene (HFO-1234yf), 1,3,3,3-tetrafluoropropene (HFO-1234ze), 1,2,3,3-tetrafluoropropene (HFO-1234ye), 1,1,2,3-tetrafluoropropene (HFO-1234yc), 1,2,3,3,3-pentafluoropropene (HFO-1225ye), 1,1,3,3,3-pentafluoropropene (HFO-1225zc) and 3,3,3-trifluoropropene (HFO-1243zf.

The haloolefin produced by a known method can be used. One such example includes a method for subjecting fluoroalkane to dehydrofluorination in the presence of a catalyst (a method described, for example, in JP-A-2012-500182). For example, when the haloolefin is tetrafluoropropene, pentafluoropropane is used as a starting material, and subjected to dehydrofluorination reaction in the presence of a catalyst to produce tetrafluoropropene. Specifically, when the haloolefin is 2,3,3,3-tetrafluoropropene (HFO-1234yf), 1,1,1,2,3-pentafluoropropane or 1,1,1,2,2-pentafluoropropane is used as a starting material, and subjected to dehydrofluorination reaction in the presence of a catalyst to produce 2,3,3,3-tetrafluoropropene (HFO-1234yf). When the haloolefin is 1,3,3,3-tetrafluoropropene (HFO-1234ze), 1,1,1,3,3-pentafluoropropane is used as a starting material, and subjected to dehydrofluorination reaction in the presence of a catalyst to produce 1,3,3,3-tetrafluoropropene (HFO-1234ze). In the above production method, chromium catalysts, such as chromium oxide or fluorinated chromium oxide, and other metal catalysts can be used as catalysts, and the reaction can be performed at a temperature in the range of 200-500°C.

When 2,3,3,3-tetrafluoropropene is produced according to the above production method, e.g. E- and Z-isomers of 1,3,3,3-tetrafluoropropene (HFO-1234ze) are produced as byproducts. In this case, the byproducts were typically removed by purifying the resulting product to obtain the target 2,3,3,3-tetrafluoropropene. However, the E- and Z-isomers of 1,3,3,3-tetrafluoropropene produced according to the above method are byproducts and, at the same time, the (b) components in the composition. Accordingly, since the (b) component, which is an essential component in the composition of this embodiment, can also be obtained in the production of 2,3,3,3-tetrafluoropropene according to the above method, 2,3,3,3-tetrafluoropropene has an advantage in that it can be used in the state containing the byproduct without purification. Of course, in the production of haloolefins other than 2,3,3,3-tetrafluoropropene according to the above method as well, as long as the byproduct is a component (b), the result can be used without purification as haloolefin in the composition. Accordingly, when the haloolefin is produced by the method in which fluoroalkane is subjected to dehydrofluorination in the presence of a catalyst, the byproduct may be contained in the haloolefin.

The component (b) in the composition is at least one compound which is different from the haloolefin (a) and is selected from HFO-1234ze, HFO-1234yf, HFC-254eb, HFC-245eb, HFC-245fa, HFC-245cb, HFC-236ea, HFC-236fa, HFC-23, HFC-32, HFC-125, HFC-143a, HFC-134a, FC-1216, HCFO-1233xf, HCFO-1233zd, HCFO-1232xf, HCFO-1223xd and chloromethane and, if the haloolefin (a) is HFO-1234ze, HFO-1234yf. These components (b) can be used singly or in a combination of two or more, and the combination is not particularly limited.

The component (b) that is produced by a known method can be used, and as described above, the component produced as a byproduct in the production of haloolefin can also be used.

Water is not particularly limited, and purified water, such as distilled water, ion exchange water, filtered water, tap water, and ultrapure water obtained by a commercially available device for generating pure water, can be used. However, since water containing acid, such as HCl, may corrode equipment or reduce the haloolefin stabilizing effect, it is preferable to remove acid, such as HCl, to an undetectable level in a typical analysis method. The amount of acid is preferably ≤ 10 mass ppm, and more preferably ≤ 1 mass ppm based on the total amount of the component (a), component (b), and component (c) in the composition.

Although the pH of the water is not particularly limited, it is generally in the range of 6-8. When the amount of acid in the water is in the above range, the pH of the water is generally within the range of 6-8.

The amount of water in the composition is 3-200 mass ppm based on the total amount of the (a) haloolefin. In this range, the haloolefin stabilizing effect is fully exhibited. The amount of water being preferably less than 30 mass ppm based on the total amount of haloolefin can easily prevent device corrosion and the acceleration of haloolefin decomposition.

When the amount of water is in this range, the stability of haloolefin in the composition is further improved. The amount of water in the composition is particularly preferably over 3 mass ppm and less than 30 mass ppm. In this range, the stability of haloolefin in the composition is further improved. The amount of water in the composition being less than 30 mass ppm inhibits prevention of refrigerant performance.

The amount of HFO-1243zf, HFO-1225ye, 3,3,3-trifluoropropine and the component (b) in the composition is 0.1 mass ppm to < 10,000 mass ppm based on the total amount of haloolefin. In this range, the haloolefin stabilizing effect may not be significantly inhibited.

The composition may contain other known additives as long as the effect of the present invention is not inhibited. The amount of other additives is preferably ≤ 50 mass%, and more preferably ≤ 40 mass% based on the total amount of the composition.

The composition can be prepared by any method. For example, each component is prepared and mixed in a predetermined composition ratio, thus obtaining a composition.

In the composition, because of the presence of water, the double bond of haloolefin is stably present, which is not likely to cause oxidization, attaining highly stable haloolefin. Accordingly, the composition can be stored for a long period of time as compared with typical haloolefins. Moreover, because of the highly stable haloolefin, the performance of haloolefin may not be significantly impaired. Accordingly, even when the composition is used as a refrigerant or a heat transfer medium, the performance of the composition as the refrigerant or heat transfer medium is excellent due to the high stability of the haloolefin.

The composition further contains (d) oxygen in an amount of ≤ 0.35 mol% based on the total amount of the (a) haloolefin. When the amount of oxygen is in this range, the stability of haloolefin in the composition is further improved. From this point of view, a lower amount of oxygen in the composition is better. However, as described above, since the composition contains water, the stability of the haloolefin can be maintained by the effect of the water, as long as the amount of oxygen is in the above range. The lower limit of the amount of oxygen in the composition is, for example, 1 ppm, which is the detection limit of gas chromatography.

When the composition is used as a refrigerant or a heat transfer medium as described above, 2,3,3,3-tetrafluoropropene (HFO-1234yf), 1,3,3,3-tetrafluoropropene (HFO-1234ze), 1,2,3,3-tetrafluoropropene (HFO-1234ye), 1,2,3,3,3-pentafluoropropene (HFO-1225ye) and 3,3,3-trifluoropropene (HFO-1243zf), are particularly advantageous as haloolefins.

The composition does not comprise polyalkyleneglycol (PAG) or polyolester (POE).

A refrigerant or heat transfer medium that mainly contains haloolefin is likely to cause decomposition or oxidization when it is in contact with e.g. metal, and is likely to lose performance as a refrigerant or heat transfer medium. However, when the above composition is used as a refrigerant or heat transfer medium, a reduction in the performance of the refrigerant or heat transfer medium can be inhibited because of the high stability of the haloolefin.

Accordingly, the composition of the present invention can be used in various applications in addition to refrigerants and heat transfer media. When the composition is used for purposes other than refrigerants and heat transfer media, examples of the haloolefin include 2,3,3,3-tetrafluoropropene (HFO-1234yf), 1,3,3,3-tetrafluoropropene (HFO-1234ze), 1,2,3,3-tetrafluoropropene (HFO-1234ye), 1,1,2,3-tetrafluoropropene (HFO-1234yc), 1,2,3,3,3-pentafluoropropene (HFO-1225ye), 1,1,3,3,3-pentafluoropropene (HFO-1225zc) and 3,3,3-trifluoropropene (HFO-1243zf).

### Examples

The present invention is explained in detail below with reference to the Examples, but the present invention is not limited to the embodiments of the Examples.

### Compositions (1a)-(1c)

2,3,3,3-Tetrafluoropropene (hereinbelow simply referred to as "HFO-1234yf") and water were prepared and mixed to produce three types of haloolefin-based compositions containing water in amounts of 10 mass ppm, 200 mass ppm, and 10,000 mass ppm relative to the HFO-1234yf, thus obtaining the compositions (1a), (1b) and (1c). The HFO-1234yf was produced, for example, by the method described in Example 1 of JP-A-2012-500182 and JP-A-2009-126803. HF generated in the above production was deoxidized by using a water washing column and an alkali column containing an NaOH aqueous solution. The resulting haloolefin-based composition might contain a byproduct (for example, 1,3,3,3-tetrafluoropropene (HFO-1234ze), i.e., component (b)) generated in the production of HFO-1234yf.

### Compositions (2a)-(2c)

1,3,3,3-Tetrafluoropropene (E-isomer, hereinbelow simply referred to as "HFO-1234ze (E)") and water were prepared and mixed to produce three types of haloolefin-based compositions containing water in amounts of 10 mass ppm, 200 mass ppm, and 10,000 mass ppm relative to the HFO-1234ze (E), , thus obtaining the compositions (2a), (2b) and (2c). The HFO-1234ze (E) was obtained together with HFO-1234yf by the dehydrofluorination of HFC-245eb according to the method described in JP-A-2012-500182. HF generated in the above production was deoxidized by using a water washing column and an alkali column containing an NaOH aqueous solution. HFO-1234yf was a (b) component.

### Composition (1d)

A haloolefin-based composition was obtained by the same method as in the case of compositions (1a)-(1c) except that water was not added.

### Composition (2d)

A haloolefin-based composition was obtained by the same method as in the case of compositions (2a)-(2c) except that water was not added.

### Haloolefin Stability Test 1

The above described haloolefin-based compositions (1a)-(1d) and (2a)-(2d) were subjected to a haloolefin stability test as described below. The haloolefin-based composition was added in a manner such that the amount of haloolefin was 0.01 mol to a glass tube (ID 8 mm Φ × OD 12 mm Φ × L 300 mm), a side of which was sealed. The tube was hermitically sealed. The tube was allowed to stand in a constant temperature bath in a 150°C atmosphere, and was kept for one week in this state. Subsequently, the tube was removed from the constant temperature bath and cooled, and then acid in the gas inside the tube was analyzed to evaluate the stability of the haloolefin.

### Haloolefin Stability Test 2

The haloolefin-based compositions obtained in the Examples and Comparative Examples were subjected to a haloolefin stability test as described below. The haloolefin-based composition was added in a manner such that the amount of haloolefin was 0.01 mol to a glass tube (ID 8 mm Φ x OD 12 mm Φ x L 300 mm), a side of which was sealed. Subsequently, oxygen was enclosed in the tube by adjusting the acid concentration to a predetermined mol concentration (0.010 mol%, 0.115 mol%, or 0.345 mol%) relative to the number of moles of haloolefin filled. The tube was allowed to stand in a constant temperature bath in a 150°C atmosphere, and was kept for one week in this state. Subsequently, the tube was removed from the constant temperature bath and cooled, and then acid in the gas inside the tube was analyzed to evaluate the stability of the haloolefin.

Acid in the gas was analyzed according to the following method. Gas remaining in the tube after cooling was completely coagulated by using liquid nitrogen. Subsequently, the tube was opened and gradually defrosted to collect gas in a sampling bag. 5 g of pure water was poured into the sampling bag, and acid was extracted into the pure water while efficiently bringing the pure water into contact with the collected gas. The extract was detected by ion chromatography, and the amounts (mass ppm) of fluoride ions (F-) and trifluoroacetate ions (CF3COO⁻) were measured.

**Table 1**

| Sampl eNo. | Invention or Compariso n | Composition | Type of Haloolefi n (HFO- ) | Amount of oxygen (mol-%) | Amount of water (mass pp m) | Amount of Acid (mass ppm) | |
|---|---|---|---|---|---|---|---|
| | | | | | | F⁻ | CF₃COO⁻ |
| 1 | Compariso n | (1d) | 1234yf | 0 | 0 (N.D.) | <1 | <1 |
| 2 | Compariso n | (1a) | | 0 | 10 | <1 | <1 |
| 3 | Compariso n | (1b) | | 0 | 200 | <1 | <1 |
| 4 | Compariso n | (1c) | | 0 | 10000 | <1 | <1 |
| 5 | Compariso n | (1d) | | 0.010 | 0 (N.D.) | 70 | 550 |
| 6 | Invention | (1a) | | 0.010 | 10 | 35 | 90 |
| 7 | Invention | (1b) | | 0.010 | 200 | 10 | 25 |
| 8 | Compariso n | (1c) | | 0.010 | 10000 | <1 | 10 |
| 9 | Compariso n | (1d) | | 0.115 | 0 (N.D.) | 300 | 1850 |
| 10 | Invention | (1a) | | 0.115 | 10 | 100 | 330 |
| 11 | Invention | (1b) | | 0.115 | 200 | 30 | 100 |
| 12 | Compariso n | (1c) | | 0.115 | 10000 | 3 | 20 |
| 13 | Compariso n | (1d) | | 0.345 | 0 (N.D.) | 1005 | 5850 |
| 14 | Invention | (1a) | | 0.345 | 10 | 330 | 1900 |
| 15 | Invention | (1b) | | 0.345 | 200 | 110 | 675 |
| 16 | Compariso n | (1c) | | 0.345 | 10000 | 50 | 275 |
| 17 | Compariso n | (2d) | 1234ze (E) | 0 | 0 (N.D.) | <1 | <1 |
| 18 | Invention | (2a) | | 0 | 10 | <1 | <1 |
| 19 | Invention | (2b) | | 0 | 200 | <1 | <1 |
| 20 | Compariso n | (2c) | | 0 | 10000 | <1 | <1 |
| 21 | Compariso n | (2d) | | 0.010 | 0 (N.D.) | 80 | 610 |
| 22 | Invention | (2a) | | 0.010 | 10 | 30 | 95 |
| 23 | Invention | (2b) | | 0.010 | 200 | 15 | 40 |
| 24 | Compariso n | (2c) | | 0.010 | 10000 | 3 | 20 |
| 25 | Compariso n | (2d) | | 0.115 | 0 (N.D.) | 410 | 1900 |
| 26 | Invention | (2a) | | 0.115 | 10 | 105 | 385 |
| 27 | Invention | (2b) | | 0.115 | 200 | 50 | 120 |
| 28 | Compariso n | (2c) | | 0.115 | 10000 | 10 | 45 |
| 29 | Compariso n | (2d) | | 0.345 | 0 (N.D.) | 1100 | 6020 |
| 30 | Invention | (2a) | | 0.345 | 10 | 335 | 1930 |
| 31 | Invention | (2b) | | 0.345 | 200 | 130 | 700 |
| 32 | Compariso n | (2c) | | 0.345 | 10000 | 55 | 290 |

The amount of oxygen in each of Samples 5-8 in Table 1 was set to 0.010 mol%. Since Sample 5 did not contain water, the amount of acid in Sample 5 was larger than those of Samples 6-8 containing water. This indicates that since the amount of acid in Sample 5 was larger, the decomposition or oxidization of HFO-1234yf, which was a haloolefin, advanced as compared to Samples 6-8. The results indicate that HFO-1234yf, which was a haloolefin, was stabilized in the samples containing water. The amount of oxygen in each of Samples 9-12 was 0.115 mol%, and the amount of oxygen in each of Samples 13-16 was 0.345 mol%; however, the results of Samples 13-16 showed a similar tendency to the results obtained when the amount of oxygen was 0.115 mol%. Further, when the haloolefin was HFO-1234ze (Samples 21-32), a similar tendency was observed. In Samples 1-4 and 17-20, the amount of acid was below 1 mass ppm, and it was found that most of the haloolefin decomposition did not proceed. This was presumably because oxygen was not added to the system, causing no oxidization. Accordingly, in the system containing substantially no oxygen, the haloolefin was always stable regardless of whether water was contained in the composition.

The above clearly indicates that water contained in the composition stabilizes haloolefin as in the present invention.

## Claims

1. A haloolefin-based composition comprising
(a) a haloolefin selected from tri-, tetra- and pentafluoropropene;
(b) at least one compound selected from HFO-1234ze, HFC-254eb, HFC-245eb, HFC-245fa, HFC-245cb, HFC-236ea, HFC-236fa, HFC-23, HFC-32, HFC-125, HFC-143a, HFC-134a, FC-1216, HCFO-1233xf, HCFO-1233zd, HCFO-1232xf, HCFO-1223xd, chloromethane and, if the haloolefin (a) is HFO-1234ze, HFO-1234yf;
(c) water; and
(d) oxygen;
wherein the amount of HFO-1243zf, HFO-1225ye, 3,3,3-trifluoropropine and the at least one compound (b)is 0.1 to < 10,000 ppm by weight based on the total amount of haloolefin,
the amount of water (c) being 3-200 ppm by weight based on the total amount of the haloolefin (a),
the amount of oxygen (d) being ≤ 0.35 mol% based on the total amount of the haloolefin (a), and
the component (b) being different from the haloolefin (a), and
wherein the composition does not comprise polyalkyleneglycol (PAG) or polyolester (POE).

2. The composition of claim 1, wherein the haloolefin (a) is tetrafluoropropene.

3. The composition of claim 2, wherein the tetrafluoropropene is 2,3,3,3-tetrafluoropropene.

4. The composition of claim 2, wherein the tetrafluoropropene is 1,3,3,3-tetrafluoropropene.

## Patentansprüche

1. Haloolefin-basierte Zusammensetzung, umfassend
(a) ein Haloolefin, ausgewählt aus Tri-, Tetra- und Pentafluorpropen;
(b) mindestens eine Verbindung, ausgewählt aus HFO-1234ze, HFC-254eb, HFC-245eb, HFC-245fa, HFC-245cb, HFC-236ea, HFC-236fa, HFC-23, HFC-32, HFC-125, HFC-143a, HFC-134a, FC-1216, HCFO-1233xf, HCFO-1233zd, HCFO-1232xf, HCFO-1223xd, Chlormethan und, falls das Haloolefin (a) HFO-1234ze ist, HFO-1234yf;
(c) Wasser; und
(d) Sauerstoff;
worin die Menge an HFO-1243zf, HFO-1225ye, 3,3,3-Trifluorpropin und der mindestens einen Verbindung (b) 0,1 bis < 10,000 ppm nach Gewicht, basierend auf der Gesamtmenge an Haloolefin, beträgt,
die Menge an Wasser (c) 3-200 ppm nach Gewicht, basierend auf der Gesamtmenge des Haloolefins (a), beträgt, und
die Menge an Sauerstoff (d) ≤ 0,35 mol%, basierend auf der Gesamtmenge des Haloolefins (a), beträgt,
der Bestandteil (b) sich von dem Haloolefin (a) unterscheidet und
worin die Zusammensetzung nicht Polyalkylenglykol (PAG) oder Polyolester (POE) umfasst.

2. Zusammensetzung gemäß Anspruch 1, worin das Haloolefin (a) Tetrafluorpropen ist.

3. Zusammensetzung gemäß Anspruch 2, worin das Tetrafluorpropen 2,3,3,3-Tetrafluorpropen ist.

4. Zusammensetzung gemäß Anspruch 2, worin das Tetrafluorpropen 1,3,3,3-Tetrafluorpropen ist.

## Revendications

1. Composition à base d'halooléfine comprenant
(a) une halooléfine sélectionnée parmi du tri-, tétra- et pentafluoropropène ;
(b) au moins un composé sélectionné parmi du HFO-1234ze, HFC-254eb, HFC-245eb, HFC-245fa, HFC-245cb, HFC-236ea, HFC-236fa, HFC-23, HFC-32, HFC-125, HFC-143a, HFC-134a, FC-1216, HCFO-1233xf, HCFO-1233zd, HCFO-1232xf, HCFO-1223xd, du chlorométhane et, si l'halooléfine (a) est du HFO-1234ze, du HFO-1234yf ;
(c) de l'eau ; et
(d) de l'oxygène ;
dans laquelle la quantité de HFO-1243zf, HFO-1225ye, 3,3,3-trifluoropropène et l'au moins un composé (b) est de 0,1 à < 10 000 ppm en poids sur la base de la quantité totale d'halooléfine,
la quantité d'eau (c) étant de 3-200 ppm en poids sur la base de la quantité totale d'halooléfine (a),
la quantité d'oxygène (d) étant ≤ 0,35 % en moles sur la base de la quantité totale de l'halooléfine (a), et
le composant (b) étant différent de l'halooléfine (a), et
dans laquelle la composition ne comprend pas de polyalkylèneglycol (PAG) ou de polyolester (POE).

2. Composition selon la revendication 1, dans laquelle l'halooléfine (a) est du tétrafluoropropène.

3. Composition selon la revendication 2, dans laquelle le tétrafluoropropène est du 2,3,3,3-tétrafluoropropène.

4. Composition selon la revendication 2, dans laquelle le tétrafluoropropène est du 1,3,3,3-tétrafluoropropène.
